# EUROPÄISCHE PATENTSCHRIFT

(11) **EP 1 082 061 B1**
(45) Veröffentlichungstag und Bekanntmachung des Hinweises auf die Patenterteilung: **10.10.2001**
(21) Anmeldenummer: 99959361.9
(22) Anmeldetag: 29.11.1999
(51) Int. Cl.: A61B 17/28

(54) **HANDGRIFF FÜR EIN MEDIZINISCHES ROHRSCHAFTINSTRUMENT**
HANDLE FOR A MEDICAL HOLLOW SHAFT INSTRUMENT
POIGNEE POUR UN INSTRUMENT MEDICAL A TIGE CREUSE

(30) Priorität: 28.12.1998 DE 19860444
(43) Veröffentlichungstag der Anmeldung: 14.03.2001
(73) Patentinhaber: Karl Storz GmbH & Co. KG, 78532 Tuttlingen (DE)
(72) Erfinder: BACHER, Uwe, D-78532 Tuttlingen (DE); CUSCHIERI, Alfred, St. Andrews, Fife K16 9TY (GB); FRANK, Tim, Wormit Newport-On-Tay, Fife DD68NG (GB)
(74) Vertreter: Heuckeroth, Volker
(86) Internationale Anmeldenummer: EP9909280
(87) Internationale Veröffentlichungsnummer: WO0038581

(56) Entgegenhaltungen:
- EP-A- 0 598 202
- WO-A-93/07816
- WO-A-97/41783
- DE-A- 19 632 135
- US-A- 2 109 147

## Beschreibung

Die Erfindung betrifft einen Handgriff für ein medizinisches Rohrschaftinstrument, mit zumindest einem beweglichen Griffteil und mit einem Kopplungsteil, durch das ein Rohrschaft mit dem Handgriff verbunden oder verbindbar ist, wobei das bewegliche Griffteil mit einem in Richtung einer Längsachse des Rohrschafts axial beweglichen Kraftübertragungselement verbunden oder verbindbar ist, wobei eine Bewegung des zumindest einen beweglichen Griffteils in eine axiale Bewegung des Kraftbertragungselements übertragen wird, und wobei das Kopplungsteil um eine quer zur Längsachse des Rohrschafts verlaufende Drehachse relativ zu einer Handgriffachse verschwenkbar und arretierbar ist. Ein derartiger Handgriff ist aus der DE 196 32 135 A1 bekannt.

Rohrschaftinstrumente werden für unterschiedlichste Arten von operativen Eingriffen am menschlichen oder tierischen Körper, insbesondere in der minimalinvasiven Chirurgie, verwendet. Solche Rohrschaftinstrumente können als Zangen, bspw. Präparier- und Faßzangen, Zangen zum Abtrennen bzw. Schneiden von Gewebe, als Stanzen oder dergleichen ausgebildet sein, wobei sich die Rohrschaftinstrumente hinsichtlich ihrer unterschiedlichen Funktion durch eine entsprechende Ausgestaltung ihrer Werkzeuge am distalen Ende unterscheiden. Als Werkzeuge am distalen Ende des Rohrschafts können Schneid-, Faß- oder Stanzwerkzeuge vorgesehen sein, wobei ein Rohrschaftinstrument zumindest ein bewegliches Werkzeug, beispielsweise in Form eines Maulteils, aufweist. Ein solches Rohrschaftinstrument kann jedoch am distalen Ende auch zwei bewegliche Werkzeuge aufweisen.

Zur Betätigung des zumindest einen beweglichen Werkzeugs weisen Rohrschaftinstrumente am proximalen Ende des Rohrschafts einen Handgriff auf. Der Rohrschaft des Rohrschaftinstruments ist üblicherweise mit einem Kopplungsteil des Handgriffs unlösbar oder lösbar verbunden. Weiterhin weist der Handgriff zumindest ein bewegliches Griffteil auf, das der eigentlichen Betätigung des zumindest einen beweglichen Werkzeugs am distalen Ende des Rohrschafts dient. Zur Betätigung des zumindest einen beweglichen Werkzeugs am distalen Ende des Rohrschafts ist das zumindest eine bewegliche Griffteil des Handgriffs über ein in Richtung der Längsachse des Rohrschafts axial bewegliches Kraftübertragungselement, beispielsweise eine Zug- oder Schubstange, mit dem zumindest einen beweglichen Werkzeug kraftschlüssig verbunden, so daß eine Bewegung des beweglichen Griffteils, bspw. eine Schwenk- oder Axialbewegung, in eine axiale Relativbewegung des Kraftübertragungselements bezüglich des Rohrschafts und schließlich in eine Bewegung des beweglichen Werkzeugs übertragen wird.

Ein Handgriff für ein Rohrschaftinstrument ist in unterschiedlichen Ausgestaltungen bekannt. In der Regel weist der Handgriff ein zweites Griffteil auf, das entweder unbeweglich und somit schaftfest oder ebenfalls beweglich ist. Ein solcher Handgriff kann weiterhin in der Art eines Scherengriffs ausgebildet sein, bei dem die beiden Griffteile seitlich von dem Rohrschaft des Rohrschaftinstruments abstehen. Bei dieser Art von Handgriffen ist das zumindest eine bewegliche Griffteil um eine quer zur Längsachse des Rohrschafts verlaufende Drehachse gelenkig mit dem anderen Griffteil verbunden. Im Sinne der vorliegenden Erfindung kann der Handgriff jedoch auch in der Art eines Pistolengriffes oder auch so ausgebildet sein, daß der Handgriff als Stabgriff in der Faust einer Hand gehalten wird.

All diesen bekannten Arten von Handgriffen ist gemeinsam, daß die Handgriffe im mit dem Rohrschaft verbundenen Zustand bezüglich dem Rohrschaft eine unveränderliche Winkelstellung einnehmen, d.h. daß eine Handgriffachse und die Längsachse des Rohrschafts einen festen Winkel miteinander bilden. Ein Handgriff, der bezüglich der Längsachse des Rohrschaftes eine unveränderliche Winkellage einnimmt, ist jedoch hinsichtlich seiner Handhabungseigenschaften nicht immer an die Anforderungen des das Rohrschaftinstrument bedienenden Arztes angepaßt. Je nach Gewohnheit werden von unterschiedlichen Ärzten unterschiedliche Griffstellungen desselben Handgriffstyps bezüglich des Rohrschaftes gewünscht. Um stets den optimal ergonomischen Handgriff zur Verfügung zu haben, wäre es daher erforderlich, für jede Art von Handgriffen einen Satz mehrerer dieser Handgriffe bereitzuhalten, die dann jeweils im mit dem Rohrschaft verbundenen Zustand eine andere Winkellage einnehmen, so daß jeder Arzt den für ihn ergonomisch optimalen Handgriff aussuchen kann. Im Fall, daß die Handgriffe nicht auswechselbar sind, bedeutet dies, daß sogar von jedem Rohrschaftinstrument ein ganzer Satz solcher Instrumente mit unterschiedlich abgewinkelten Handgriffen bereitgehalten werden muß.

Andererseits kann es im Einzelfall auch wünschenswert oder erforderlich sein, daß der Handgriff bei ein und demselben Rohrschaftinstrument während eines Operationsvorgangs unterschiedliche Winkelstellungen bezüglich des Rohrschafts einnehmen kann, um bei einem operativen Eingriff die jeweils bequemste Handstellung für den gerade durchzuführenden Operationsvorgang zu haben. Bei den bekannten Handgriffen, von denen jeweils ein ganzer Satz vorhanden wäre, würde dies bedeuten, daß während des Operationsvorgangs mehrmals der Handgriff ausgetauscht oder bei abnehmbaren Handgriffen sogar das ganze Instrument ausgetauscht werden müßte, was die Operationsdauer wesentlich verlängert.

Die bekannten Handgriffe erweisen sich demnach hinsichtlich ihrer ergonomischen Eigenschaften als nachteilig.

Demgegenüber weist das aus der eingangs genannten DE 196 32 135 A1 bekannte Instrument einen abwinkelbaren Handgriff auf, so daß die ergonomischen Eigenschaften dieses bekannten Handgriffs bzw. des damit ausgestatteten Instruments gegenüber den zuvor genannten bekannten Handgriffen und Instrumenten verbessert ist.

Die Übertragung der Bewegung des beweglichen Griffteils auf das Kraftübertragungselement im Rohrschaft erfolgt bei diesem bekannten Handgriff durch eine an das bewegliche Griffteil angeschweißte Exzenterscheibe. Die Exzenterscheibe wirkt unter Reibung mit einem Stößel zusammen.

Der Erfindung liegt daher die Aufgabe zugrunde, für die Kraftübertragung vom beweglichen Griffteil auf das Kraftübertragungselement bei einem verschwenkbaren Handgriff einen alternativen Mechanismus anzugeben.

Hinsichtlich des eingangs genannten Handgriffs wird diese Aufgabe erfindungsgemäß dadurch gelöst, daß ein als zweiarmiger Hebel ausgebildetes Steuerelement vorgesehen ist, dessen einer Hebelarm mit dem beweglichen Griffteil und dessen anderer Hebelarm mit dem Kraftübertragungselement in Verbindung steht, derart, daß eine Bewegung des beweglichen Griffteils eine Drehung des Steuerelements bewirkt, die vom Steuerelement in eine axiale Bewegung des Kraftübertragungselements umgesetzt wird.

Erfindungsgemäß ist demnach eine Kraftübertragungsmechanik zwischen dem beweglichen Griffteil und dem Kraftübertragungselement vorgesehen, bei der zwischen diese beiden Teile ein Steuerelement geschaltet ist, dessen einer Hebelarm mit dem beweglichen Griffteil und dessen anderer Hebelarm mit dem Kraftübertragungselement in Verbindung steht. Das bewegliche Griffteil greift dazu an dem einen Hebelarm des Steuerelements und das Kraftübertragungselement am anderen Hebelarm des Steuerelements an, wobei eine Betätigung des beweglichen Griffteils eine Drehung des Steuerelements bewirkt, die vom Steuerelement über den zweiten Hebelarm in eine axiale Bewegung des Kraftübertragungselements umgesetzt wird. Diese Art der Kraftübertragungsmechanik von dem beweglichen Griffteil auf das Kraftübertragungselement ermöglicht eine von der jeweils eingestellten Schwenkstellung des Kopplungsteils und damit von der jeweils eingestellten Schwenkstellung des Handgriffs, unabhängige Kraftübertragung, wobei der weitere Vorteil erzielt wird, daß die Verhältnisse der Kraftübertragung in jeder Schwenkstellung des Handgriffs bezüglich des Rohrschafts gleich groß gewählt werden können. Dadurch wird vermieden, daß zur Betätigung des Werkzeugs am distalen Ende des Rohrschafts in Abhängigkeit von der Schwenkstellung des Handgriffs unterschiedliche Kräfte vom bedienenden Arzt aufgebracht werden müssen.

Somit wird die der Erfindung zugrunde liegende Aufgabe vollkommen gelöst.

In einer bevorzugten Ausgestaltung fällt die Drehachse des Kopplungsteils mit einer Drehachse des Steuerelements zusammen.

Hierdurch wird ein einfacher Aufbau der Kraftübertragungsmechanik erreicht, da für das Kopplungsteil und für das Steuerelement nur eine einzige Drehlagerung vorgesehen werden muß.

In einer weiteren bevorzugten Ausgestaltung ist das Steuerelement mit dem Kopplungsteil verbunden und wird mit diesem bei einem Verschwenken des Kopplungsteils zum Abwinkeln des Handgriffs mit verschwenkt, wobei zum Verschwenken des Steuerelements die Verbindung zwischen dem beweglichen Griffteil lösbar und nach dem Verschwenken wieder herstellbar ist.

Diese Maßnahme hat den Vorteil, daß dann, wenn das Kopplungsteil verschwenkt wird, um den Handgriff bezüglich der Längsachse des Rohrschafts in eine andere Winkelstellung zu bringen, gleichzeitig auch das Steuerelement mit verschwenkt wird, so daß die Lage des Steuerelements an die neu eingestellte Winkelstellung des Handgriffs angepaßt werden kann, wodurch eine Veränderung der Kraftübertragung vermieden wird, ohne daß das Steuerelement dazu zusätzlich manipuliert werden muß.

In einer weiteren bevorzugten Ausgestaltung ist das zumindest eine bewegliche Griffteil mit einem ersten Hebelarm eines zweiarmigen Hebels verbunden, dessen anderer Hebelarm mit dem zweiten Hebelarm des Steuerelements verbunden ist, wobei eine Drehachse des Hebels mit der Drehachse des Kopplungsteils zusammenfällt.

Durch diese Maßnahme wird eine kinematisch günstige, eine in allen Winkelstellungen hohe Kraftübertragung ermöglichende Kraftübertragung von dem beweglichen Griffteil auf das Steuerelement erreicht.

Dabei ist es bevorzugt, wenn der zweite Hebelarm des Hebels über einen Zapfen mit dem Steuerelement verbunden ist, wobei in dem Steuerelement mehrere auf einem Teilkreis angeordnete Bohrungen vorhanden sind, mit denen der Zapfen je nach Schwenkstellung des Kopplungsteils in Eingriff bringbar ist.

Diese Maßnahme ist insbesondere mit der zuvor erwähnten Maßnahme, daß das Steuerelement zusammen mit dem Kopplungsteil verschwenkbar ausgebildet ist, von Vorteil, weil der zuvor erwähnte Hebel in jeder eingestellten Schwenkstellung des Kopplungsteils vor dem Verschwenken mit dem Steuerelement leicht außer Eingriff bringbar und nach dem Verschwenken wieder in Eingriff bringbar ist.

In einer weiteren bevorzugten Ausgestaltung ist das Kopplungsteil mittels eines Rastmechanismus in mehreren Schwenkstellungen in einem Gesamtwinkelbereich von bis zu etwa 240° bezüglich der Längsachse des Rohrschafts arretierbar.

Gemäß dieser Maßnahme kann demnach der Handgriff in einem Winkelbereich zwischen etwa -120° und etwa +120° verschwenkt und in zumindest zwei, vorzugsweise jedoch einer Vielzahl von Schwenkstellungen, arretiert werden, wodurch die Ergonomie des Handgriffs hinsichtlich seiner Winkelstellung bezüglich des Rohrschafts über einen großen Winkelbereich an die gewünschten Anforderungen des Arztes anpaßbar ist. Der Schwenkbereich kann jedoch auch kleiner sein, bspw. zwischen etwa-90° und +90° liegen, oder zwischen etwa 0° und +120°, um nur einige Beispiele zu nennen.

Dabei ist es bevorzugt, wenn der Rastmechanismus eine Rastnase aufweist, die mit Aussparungen des Kopplungsteils in Eingriff bringbar ist, wobei der Rastmechanismus weiterhin einen Druckknopf aufweist, so daß durch Drücken des Druckknopfs die Rastnase mit der jeweiligen Aussparung außer Eingriff bringbar ist.

Diese Art der Arretierung bzw. Entarretierung des Kopplungsteils führt vorteilhafterweise zu einer leicht bedienbaren Verschwenkbarkeit und Arretierbarkeit des Handgriffs.

In einer weiteren bevorzugten Ausgestaltung ist der Zapfen mit dem Druckknopf verbunden und beim Drücken des Druckknopfs mit der jeweiligen Bohrung des Steuerelements außer Eingriff bringbar.

Durch diese Maßnahme wird sowohl der konstruktive Aufbau des Handgriffs vereinfacht als auch die Bedienungsfreundlichkeit verbessert, weil nur ein Druckknopf betätigt werden muß, um sowohl das Kopplungsteil als auch das Steuerelement zum Verschwenken zu entarretieren.

In einer weiteren bevorzugten Ausgestaltung weist der zweite Hebelarm des Steuerelements eine als Steuerkurve ausgebildete Aussparung auf, in der ein mit dem Kraftübertragungselement verbundener Stift axial geführt ist.

Durch diese Ausgestaltung des Steuerelements mit einer Steuerkurve, die entsprechend geformt ist, wird auf konstruktiv besonders einfache Weise eine Umsetzung der Drehbwegung des Steuerelements in eine Axialbewegung des Kraftübertragungselements erreicht.

Alternativ dazu kann das Kraftübertragungselement auch über einen Hebel mit dem Steuerelement verbunden sein.

Bei dieser Ausgestaltung ist der Hebel dann mit einem Ende am Steuerelement angelenkt, wobei eine Drehung des Steuerelements dann den Hebel auslenkt und die Auslenkung des Hebels dann bspw. in der Art eines Pleuels eine Translationsbewegung des Kraftübertragungselements hervorruft.

In einer weiteren bevorzugten Ausgestaltung weist das Kopplungsteil eine Aufnahme für ein proximales Ende des Kraftübertragungselements auf, wobei die Aufnahme axial beweglich ist und mit dem Stift bzw. mit dem Hebel verbunden ist.

Bei dieser Ausgestaltung ist das Kraftübertragungselement demnach nicht direkt mit dem Steuerelement verbunden, sondern über eine außerhalb des Steuerelements liegende Aufnahme, wodurch der Vorteil erreicht wird, daß sich die Verbindung zwischen dem Kraftübertragungselement und dem Steuerelement besonders leicht herstellen läßt. Außerdem eröffnet diese Ausgestaltung die Möglichkeit, das Kraftübertragungselement lösbar mit dem Handgriff zu verbinden.

Dabei ist es bevorzugt, wenn die Aufnahme einen Verrastmechanismus zum lösbaren Verbinden des Kraftübertragungselements mit dem Kopplungsteil aufweist.

Hierdurch wird der Vorteil erzielt, daß sich das Kraftübertragungselement von dem Kopplungsteil und damit von dem Handgriff abnehmen läßt, wodurch das Kraftübertragungselement nach dem Abnehmen von dem Handgriff leichter gereinigt werden kann.

In einer weiteren bevorzugten Ausgestaltung weist das Kopplungsteil eine Aufnahme für den Rohrschaft zum lösbaren Verbinden des Rohrschafts mit dem Kopplungsteil auf.

Hierbei ist von Vorteil, daß auch der Rohrschaft vom Handgriff abgenommen werden kann, wodurch die Reinigungseigenschaften eines derartigen Rohrschaftinstruments weiter verbessert sind.

Ein erfindungsgemäßes Rohrschaftinstrument, das einen Rohrschaft, zumindest ein bewegliches Werkzeug am distalen Ende des Rohrschafts und ein axial in dem Rohrschaft beweglich angeordnetes Kraftübertragungselement zum Betätigen des zumindest einen beweglichen Werkzeugs aufweist, ist vorteilhafterweise mit einem erfindungsgemäßen Handgriff mit einem oder mehreren der zuvor genannten Merkmale ausgestattet.

Dabei ist es bevorzugt, wenn der Rohrschaft und/oder das Kraftübertragungselement lösbar mit dem Handgriff verbunden ist/ sind, und/oder wenn außerdem das Kraftübertragungselement lösbar mit dem Rohrschaft verbunden ist.

Durch die lösbare Ausgestaltung sowohl des Rohrschafts als auch des Kraftübertragungselements kann das Rohrschaftinstrument in den Rohrschaft, das Kraftübertragungselement und den Handgriff zerlegt werden, wodurch sich jede dieser Baugruppen besser reinigen läßt.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird hiernach mit Bezug auf diese näher beschrieben. Es zeigen:
- Fig. 1: eine perspektivische Gesamtansicht eines Rohrschaftinstruments, das mit einem erfindungsgemäßen Handgriff ausgestattet ist;
- Fig. 2: den Handgriff in Fig. 1 in einer teilweise geschnittenen perspektivischen Ansicht, wobei gegenüber Fig. 1 Teile weggelassen wurden;
- Fig. 3: den Handgriff in einer perspektivischen Darstellung auf die in Figuren 1 und 2 verdeckte Seite, mit weiteren Weglassungen von Teilen;
- Fig. 4: eine schematische Prinzipdarstellung des Kraftübertragungsmechanismus in einer ersten Betriebsstellung, in der die Handgriffachse mit der Längsachse des Rohrschafts einen Winkel von etwa 0° bildet; und
- Fig. 5: eine schematische Prinzipdarstellung des Kraftübertragungsmechanismus in einem Betriebszustand, in dem die Handgriffachse mit der Längsachse des Rohrschafts einen Winkel von etwa 60° bildet.

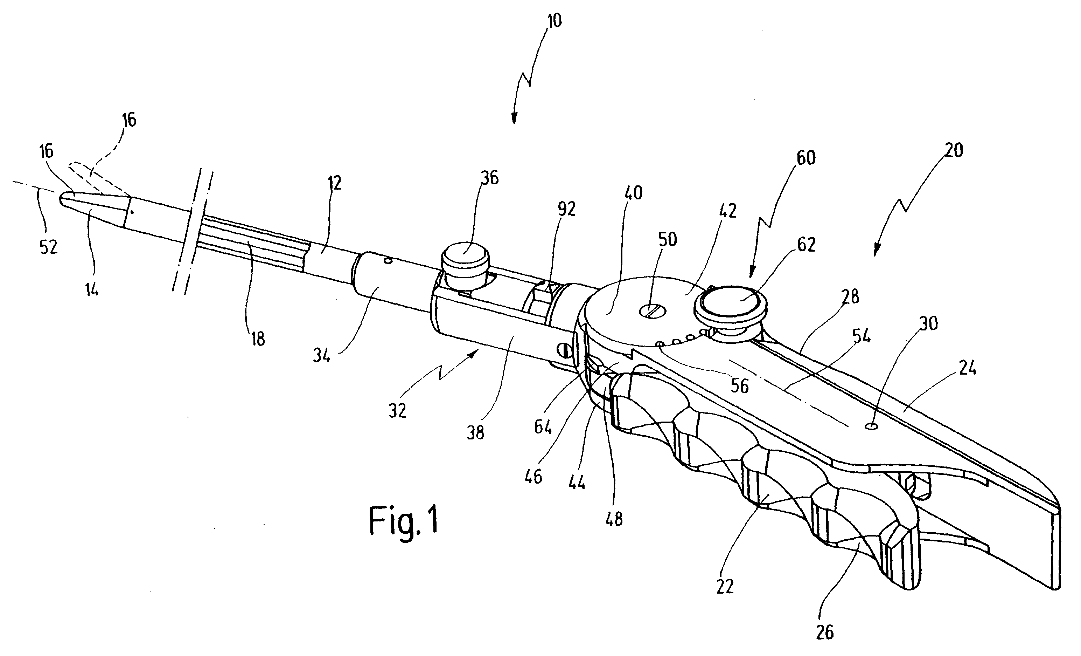

In Fig. 1 ist ein mit dem allgemeinen Bezugszeichen 10 versehenes medizinisches Rohrschaftinstrument dargestellt, das für einen operativen Eingriff im menschlichen oder tierischen Körper zum Präparieren von Gewebe verwendet werden kann.

Das Rohrschaftinstrument 10 weist einen lang erstreckten Rohrschaft 12 auf, an dessen distalen Ende ein erstes Werkzeug 14 und ein zweites Werkzeug 16 angeordnet sind. Das zweite Werkzeug 16 ist relativ zu dem ersten Werkzeug 14 beweglich ausgestaltet und zu diesem Zweck gelenkig mit dem Rohrschaft 12 verbunden.

Das erste Werkzeug 14 und das zweite Werkzeug 16 sind als Maulteile ausgebildet, die eine Schneidfunktion zum Abtrennen von Gewebe aufweisen.

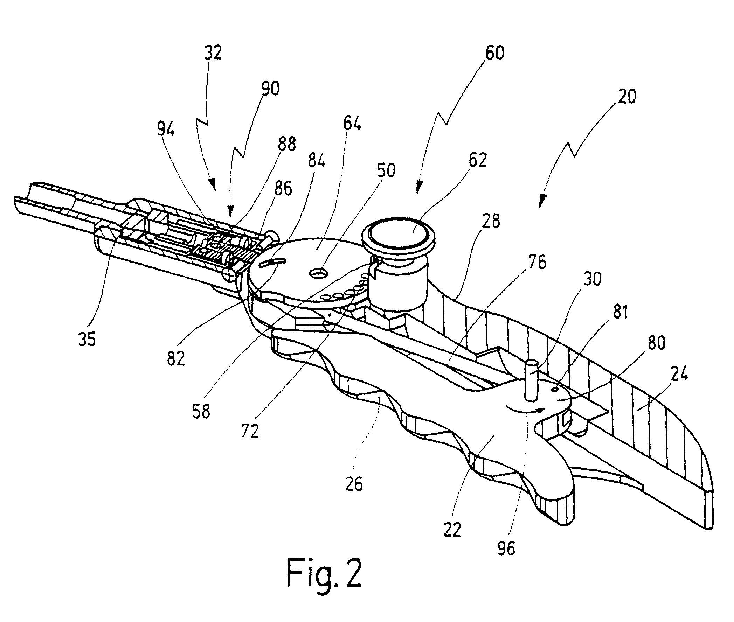

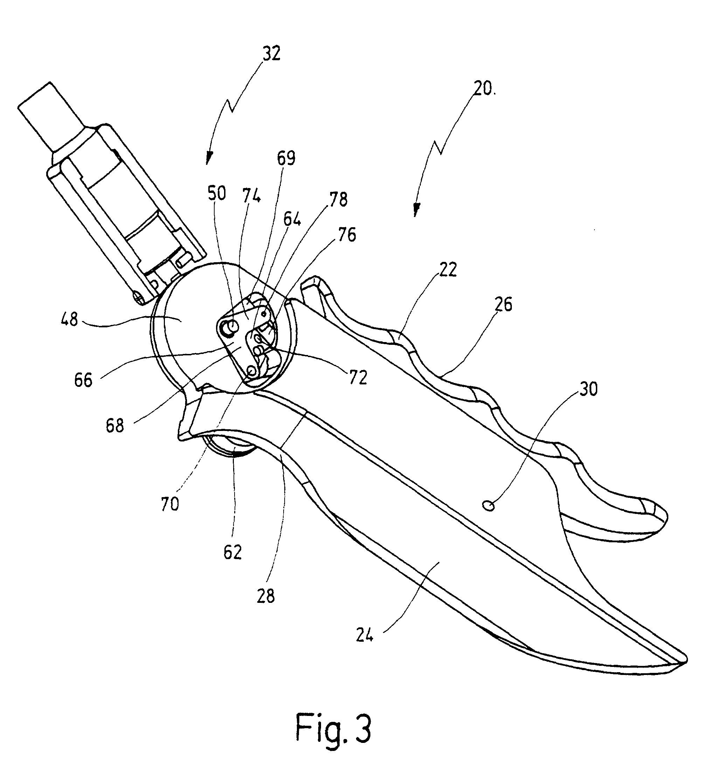

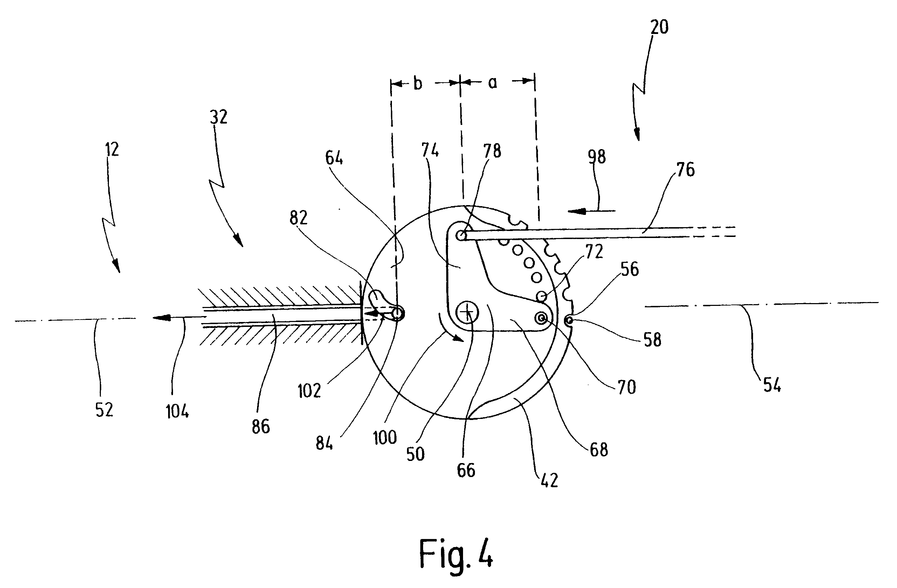

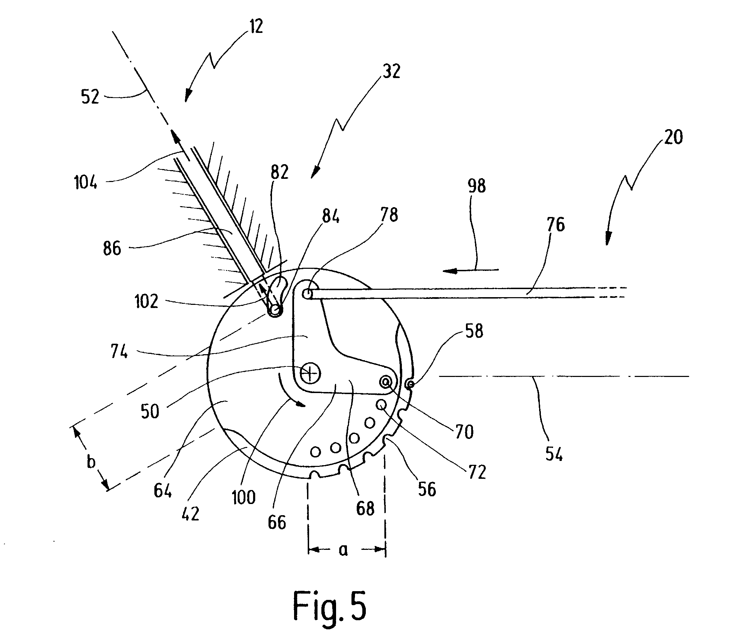

Am proximalen Ende des Rohrschafts 12 weist das Rohrschaftinstrument 10 einen Handgriff 20 auf, der hiernach mit Bezug auf Fig. 1 sowie mit Bezug auf Figuren 2 und 3 näher beschrieben wird. Ferner zeigen Figuren 4 und 5 schematische Prinzipskizzen der Funktionsweise der Kraftübertragungsmechanik des Handgriffs 20.

Gemäß Figuren 1 bis 3 weist der Handgriff 20 ein bewegliches Griffteil 22 und ein unbewegliches Griffteil 24 auf. Das bewegliche Griffteil 22 weist vier Fingermulden 26 für Zeige-, Mittel-, Ring- und den kleinen Finger auf. Das unbewegliche Griffteil 24 weist entsprechend eine Daumenmulde 28 für den Daumen auf.

Das bewegliche Griffteil 22 ist über eine Drehachse 30 gelenkig mit dem unbeweglichen Griffteil 24 verbunden.

Der Handgriff 20 weist weiterhin an seinem distalen Ende ein Kopplungsteil 32 auf, durch das der Rohrschaft 12 mit dem Handgriff 20 verbunden bzw. verbindbar ist. Dazu ist ein proximales Ende des Rohrschafts 12 in ein distales Rohrstück 34 des Kopplungsteils 32 eingeschoben und mit dem Kopplungsteil 32 über einen Rastmechanismus bei 35 (vgl. Fig. 2) verrastet, der über einen Druckknopf 36 wieder lösbar ist, um den Rohrschaft 12 von dem Kopplungsteil 32 abnehmen zu können.

Das Rohrstück 34 ist über eine Gabel 38, die einen Mittelabschnitt des Kopplungsteils 32 bildet, mit einem proximalen Endabschnitt 40 des Kopplungsteils 32 starr verbunden, das ebenfalls in Form einer Gabel ausgebildet ist, die aus zwei im wesentlichen runden Platten 42 und 44 gebildet ist. Der gabelförmige Endabschnitt 40, d.h. genauer die Platte 42 und die Platte 44, sitzen jeweils auf einem Gabelschenkel 46 bzw. 48 des unbeweglichen Griffteils 24, so daß das Kopplungsteil 32 mit dem unbeweglichen Griffteil 24 verbunden ist.

Die Besonderheit des Kopplungsteils 32 besteht nun darin, daß der Endabschnitt 40 des Kopplungsteils 32 um eine Drehachse 50, die quer zu einer Längsachse 52 des Rohrschafts 12, im vorliegenden Ausführungsbeispiel senkrecht zur Längsachse 52 verläuft, schwenkbar ist. Dadurch kann der Handgriff 20 bezüglich des Rohrschafts 12 in verschiedenen Winkelstellungen abgewinkelt werden. Eine Handgriffachse 54 des Handgriffs 20, die hier etwa als Mittelachse des Handgriffs 20 gewählt ist, kann somit bezüglich der Längsachse 52 des Rohrschafts 12 verschiedene Winkelstellungen einnehmen, und zwar Winkelstellungen zwischen etwa 0°(vgl. Fig. 4) und etwa 60° (vgl. Fig. 5), so daß bei dem gezeigten Ausführungsbeispiel die maximale Winkelversatzstellung zwischen der Handgriffachse 54 und der Längsachse 52 des Rohrschafts 12 etwa 60° beträgt . Es kann jedoch für andere Rohrschaftinstrumente, an denen der Handgriff 20 verwendet wird, sinnvoll sein, das Kopplungsteil über einen Gesamtwinkelbereich von etwa 240°, bspw. zwischen -120° und +120° verschwenkbar und arretierbar auszugestalten.

Das Kopplungsteil 32 ist in mehreren Winkelstellungen, bei dem vorliegenden Ausführungsbeispiel in sechs verschiedenen Winkelstellungen, arretierbar. Dazu weist die Platte 42 des Endabschnitts 40 umfänglich angeordnete Aussparungen 56 in Form von Einkerbungen auf, in die eine Rastnase 58 eingreift.

Die Aussparungen 56 und die Rastnase 58 sind Teil eines Rastmechanismus 60, der weiterhin einen Druckknopf 62 aufweist, mit dem die Rastnase 58 fest verbunden ist. Durch Herunterdrücken des Druckknopfes 62 wird die Rastnase 58 mit der Aussparung 56, in die die Rastnase 58 gerade eingreift, außer Eingriff gebracht, wonach sich das Kopplungsteil 32 um die Drehachse 50 bezüglich der Griffteile 22 und 24 verschwenken läßt. Durch Loslassen des Druckknopfs 62, der ortsfest an dem unbeweglichen Griffteil 24 befestigt ist, rückt die Rastnase 58 in die je nach Schwenkstellung auf Höhe der Rastnase 58 befindliche Aussparung 56 selbsttätig ein, wodurch das Kopplungsteil 32 drehfest an dem unbeweglichen Griffteil 24 und dem beweglichen Griffteil 22 arretiert ist.

Der Druckknopf 62 ist vorzugsweise gefedert ausgebildet, so daß er gegen eine Federkraft eindrückbar ist und nach Loslassen selbsttätig in seine Ausgangsstellung zurückkehrt.

Der Handgriff 20 weist ferner eine Kraftübertragungsmechanik zur Kraftübertragung von dem beweglichen Griffteil 22 auf das Kraftübertragungselement 18 auf, wie im folgenden näher beschrieben wird.

Die Kraftübertragungsmechanik weist ein Steuerelement 64 auf.

Das Steuerelement 64 ist zwischen den Platten 42 und 44 des gabelförmigen Endabschnitts 40 des Kopplungsteils 32 angeordnet.

Das Steuerelement 64 ist in Form einer im wesentlichen runden Platte ausgebildet, die um die gleiche Drehachse 50 wie das Kopplungsteil 32 drehbar gelagert ist. Die Drehachse 50 wird dabei durch einen durch die Platte 42, die distalen Gabelschenkel 46 und 48 des unbeweglichen Griffteils 24, das Steuerelement 64 und die Platte 44 durchgehenden Zapfen gebildet, der in Form einer Schraube ausgebildet ist, wobei die Schraube die vorgenannten Teile zusammenhält.

Das Steuerelement 64 ist dabei relativ zu den Platten 42 und 44 des gabelförmigen Endabschnitts 40 des Kopplungsteils 32 um die Drehachse 50 verdrehbar.

Das Steuerelement 64 ist zwischen den distalen Gabelschenkeln 46 und 48 des unbeweglichen Griffteils 24 angeordnet. Das Steuerelement 64 ist ferner als zweiarmiger Hebel ausgebildet, wie hiernach noch näher beschrieben wird.

Das Steuerelement 64 wirkt mit einem zweiarmigen Hebel 66 zusammen, der in einer Ausnehmung 69, die sich auf der dem Steuerelement 64 abgewandten Seite des Gabelschenkels 48 des unbeweglichen Griffteils 24 befindet, aufgenommen ist.

Der Hebel 66 ist in der Ausnehmung 69 verschwenkbar gelagert, und zwar um dieselbe Drehachse 50 wie das Kopplungsteil 32 und das Steuerelement 64, d.h. die zuvor erwähnte Schraube geht auch durch den Hebel 66 durch.

Der Hebel 66 weist einen ersten Hebelarm 68 auf, der mit dem Steuerelement 64 über einen Zapfen 70 kraftschlüssig verbunden ist, wobei der Zapfen 70 einerseits in eine Bohrung am äußeren Ende des ersten Hebelarms 68 des Hebels 66 sowie in eine von mehreren Bohrungen 72 eingreift, die umfänglich über einen Teilkreis verteilt in dem Steuerelement 64 vorhanden sind. Die Anzahl der Bohrungen 72 ist dabei gleich der Anzahl der Aussparungen 56 in dem gegabelten Endabschnitt 40, genauer in der Platte 42 desselben.

Der Zapfen 70 ist weiterhin mit dem Druckknopf 62 des Rastmechanismus 60 verbunden, und zwar derart, daß beim Herunterdrücken des Druckknopfs 62 der Zapfen 70 zwar mit dem ersten Hebelarm 68 des Hebels 66 in Eingriff bleibt, jedoch mit der entsprechenden Bohrung 72, in die er zuvor eingegriffen hat, außer Eingriff gebracht wird. Dadurch wird bewirkt, daß beim Herunterdrücken des Druckknopfs 62 sowohl das Kopplungsteil 32 als auch das Steuerelement 64, das mit dem Kopplungsteil 32 verbunden ist, wie hiernach noch beschrieben wird, gemeinsam um die Drehachse 50 verschwenkt werden, wogegen der Hebel 66 an der Schwenkstellung nicht teilnimmt.

Der Zapfen 70 ist ferner an dem Druckknopf verschwenkbar gelagert, um der Drehbewegung des Hebels 66 folgen zu können.

Der Hebel 66, der in Form eines Winkels ausgebildet ist, weist ferner einen zweiten Hebelarm 74 auf, der über eine Koppelstange 76, die einerseits an dem zweiten Hebelarm 74 bei 78 angelenkt ist, mit dem beweglichen Griffteil 22 verbunden ist, indem die Koppelstange 76 andererseits an einem Fortsatz 80 des beweglichen Griffteils 22 an einem von der Drehachse 30 beabstandeten Anlenkungspunkt 81 angelenkt ist.

Das Steuerelement 64 weist in einem den Bohrungen 72 gegenüberliegenden Bereich eine als Steuerkurve ausgebildete Aussparung 82 auf. Die durch die Aussparung 82 gebildete Steuerkurve weist einen gekrümmten Verlauf auf, wie in Figuren 4 und 5 dargestellt ist. In die Aussparung 82 greift ein Stift 84 ein, der in der Aussparung 82 axial beweglich geführt ist. Der Stift 84 sitzt am proximalen Ende eines axial in dem Kopplungsteil 32 verschiebbar aufgenommenen stabförmigen Elements 86, an dessen distalen Ende eine Aufnahme 88 in Form einer Kugelpfanne für ein proximales Ende des in Fig. 2 nicht dargestellten Kraftübertragungselements 18 angeordnet ist. Das proximale Ende des Kraftübertragungselements 18 ist in der Aufnahme 88 des Kopplungsteils 32 mittels eines Rastmechanismus 90, der einen Druckknopf 92 aufweist, lösbar verrastet.

Das stabförmige Element 86 und damit die Aufnahme 88 sind über eine in einem Federsitz 94 sitzende, nicht dargestellte Druckfeder nach proximal vorgespannt.

Im folgenden wird nun die Funktionsweise des Handgriffs 20, genauer gesagt der Kraftübertragungsmechanik von dem beweglichen Griffteil 22 auf das Kraftübertragungselement 18, näher beschrieben.

Dazu ist in Fig. 4 eine erste Betriebsstellung des Handgriffs 20 dargestellt, in der die Handgriffachse 54 mit der Längsachse 52 des Rohrschafts 12 fluchtet. Im einsatzbereiten Zustand des Handgriffs 20 ist die Rastnase 58 des Rastmechanismus 60 in die entsprechende Aussparung 56 der Platte 42 des gabelförmigen Endabschnitts 40 des Kopplungsteils 32 eingerastet, so daß der Handgriff 20 starr, d.h. drehfest, mit dem Rohrschaft 12 verbunden ist. Weiterhin ist der Zapfen 70 in die entsprechende Bohrung 72 des Steuerelements 64 eingerückt, so daß der Hebel 66, genauer der erste Hebelarm 68, kraftschlüssig mit dem Steuerelement 64 verbunden ist.

Wird nun das bewegliche Griffteil 22 um die Drehachse 30 gemäß Fig. 2 in Richtung eines Pfeiles 96 verschwenkt, wird die Koppelstange 76 gemäß Fig. 4 axial in Richtung eines Pfeiles 98 nach distal verschoben. Da die Koppelstange 76 mit ihrem anderen Ende an dem zweiten Hebelarm 74 des Hebels 66 an dem Anlenkungspunkt 78 angreift, wird der Hebel 66 um die Drehachse 50 gemäß einem Pfeil 100 in Fig. 4 verschwenkt. Der einstückig mit dem zweiten Hebelarm 74 verbundene erste Hebelarm 68 überträgt die Drehbewegung des Hebels 66 in eine gleichsinnige Verdrehung des Steuerelements 64, d.h. ebenfalls in Richtung des Pfeiles 100.

Die Strecke zwischen dem Zapfen 70 bzw. der Bohrung 72 und der Drehachse 50 bildet somit einen ersten Hebelarm a des Steuerelements 64. Ein zweiter Hebelarm b des Steuerelements 64 wird durch die Strecke zwischen der Drehachse 50 und der Aussparung 82 in dem Steuerelement 64 gebildet.

Durch die Verdrehung des Steuerelements 64 um die Drehachse 50 wird nun der Stift 84 in der als Steuerkurve ausgebildeten Aussparung 82 axial in Richtung eines Pfeiles 102 nach distal verschoben, wodurch auch das stabförmige Element 86 und über dieses das Kraftübertragungselement 18 in Fig. 1 nach distal, d.h. in Richtung eines Pfeiles 104, verschoben wird, wodurch das bewegliche Maulteil 16 bewegt wird. Die Drehbewegung des Steuerelements 64 um die Drehachse 50, die sich über einen kleinen Drehwinkel von etwa 5 bis 10° vollzieht, wird demnach in eine axiale Bewegung des Kraftübertragungselements 18 umgesetzt. Bei dieser durch die Betätigung des beweglichen Griffteils 22 vermittelten Drehbewegung des Steuerelements 64 bleibt das Kopplungsteil 32 bezüglich der Drehachse 50 drehfest, d.h. das Kopplungsteil 32 wird nicht mitgedreht. Nach Loslassen des beweglichen Griffteils 22 erfolgen die zuvor genannten Bewegungsabläufe jeweils in umgekehrter Richtung selbsttätig, was durch die Federbeaufschlagung des stabförmigen Elements 86 in proximale Richtung bewirkt wird.

Anstelle der als Steuerkurve ausgebildeten Aussparung 82 und des Stifts 84, der in der Ausparung 82 geführt ist, kann auch die Verbindung zwischen dem Kraftübertragungselement 18 und dem Steuerelement 64 durch einen nicht dargestellten Hebel bewerkstelligt werden, dessen eines Ende an dem Steuerelement 64 angelenkt ist, und dessen anderes Ende mit der Aufnahme 88 verbunden ist. Eine Verdrehung des Steuerelements 64 lenkt dann das erste Ende des Hebels aus, wodurch das andere mit der Aufnahme 88 verbundene Ende in der Art eines Pleuels axial verschoben wird, d.h. eine Translationsbewegung ausführt.

Soll nun der Handgriff 20 eine andere Winkelstellung bezüglich des Rohrschafts 12 einnehmen, wird der Druckknopf 62 des Rastmechanismus 60 gedrückt, wodurch die Rastnase 58 mit der Aussparung 56 in der Platte 42 des Kopplungsteils 32 sowie der Zapfen 70 mit der Bohrung 72 des Steuerelements 64 außer Eingriff gebracht wird. Bei gedrücktem Druckknopf 62 läßt sich nun das bewegliche Griffteil 22 zusammen mit dem unbeweglichen Griffteil 24 bezüglich des Rohrschafts 12 in dem gewünschten Winkel abwinkeln.

In Fig. 5 ist eine maximale abgewinkelte Stellung des Rohrschafts 12 bezüglich des Handgriffs 20 dargestellt, in der die Längsachse 52 des Rohrschafts 12 gegenüber der Handgriffachse 54 des Handgriffs 20 um etwa 60° abgewinkelt ist.

Nach Loslassen des Druckknopfes 62 rasten die Rastnase 58 und der Zapfen 70 selbsttätig in die Aussparung 56 bzw. in die Bohrung 72 wie zuvor beschrieben ein.

Wie aus Fig. 5 hervorgeht, hat sich die Lage des Hebels 66 bezüglich der Drehachse 50 dabei nicht verändert. Das Steuerelement 64 und das Kopplungsteil 32 wurden dagegen gemeinsam um die Drehachse 50 verschwenkt. Wird nun wieder das Griffteil 22 gemäß Fig. 2 in Richtung des Pfeiles 96 um die Drehachse 30 verschwenkt, bewirkt dies wiederum eine Axialbewegung der Koppelstange 76, die eine Verschwenkung des Hebels 66 um die Drehachse 50 gemäß dem Pfeil 100 und gleichzeitig damit eine Verdrehung des Steuerelements 64 ebenfalls in Richtung des Pfeiles 100 bewirkt, wobei diese Verdrehung des Steuerelements 64 wiederum in eine axiale Verschiebung des Stifts 84 in der Aussparung 82 des Steuerelements 64 und damit in eine axiale Bewegung des Kraftübertragungselements 18 gemäß dem Pfeil 104 umgesetzt wird.

Aus Figuren 4 und 5 geht hervor, daß sich das Kraftübertragungsverhältnis durch die veränderte Winkelstellung des Handgriffs 20 bezüglich des Rohrschafts 12 nicht verändert hat, weil der Hebelarm a und der Hebelarm b die gleiche Länge wie in der Betriebsstellung gemäß Fig. 4 aufweisen, so daß das bewegliche Griffteil 22 in beiden Betriebsstellungen gemäß Figuren 4 und 5 nicht mit jeweils unterschiedlicher Kraft betätigt werden muß, um das bewegliche Werkzeug 16, beispielsweise zum Abtrennen von Gewebe, zu betätigen.

Ferner ist das Rohrschaftinstrument 10 gemäß Fig.1 in den Handgriff 20, den Rohrschaft 12 und das Kraftübertragungselement 18 zerlegbar. Die lösbare Verbindung zwischen dem Rohrschaft 12 und dem Handgriff 20 sowie zwischen dem Kraftübertragungselement 18 und dem Handgriff 20 wurde vorstehend bereits beschrieben. Ferner ist jedoch auch das Kraftübertragungselement 18 von dem Rohrschaft 12abnehmbar, indem diese beiden Teile mittels eines bajonettartigen Verschlusses im Bereich der Werkzeuge 14, 16 miteinander lösbar verbunden sind

Während das Kraftübertragungselement 18 bei dem zuvor beschriebenen Ausführungsbeispiel auf Schub arbeitet, kann der erfindungsgemäße Kraftübertragungsmechanismus mit geringfügigen Modifikationen, indem die Steuerkurve der Aussparung 82 in dem Steuerelement 64 umgekehrt zu der zuvor beschriebenen Ausgestaltung orientiert wird, leicht auf den Fall angepaßt werden, daß das Kraftübertragungselement 18 auf Zug arbeitet. Ebenso kann die Kraftübertragung von dem beweglichen Griffteil 22 auf das Steuerelement 64 anstatt, wie zuvor beschrieben, durch eine Schubbewegung der Koppelstange 76 auch durch eine Zugbewegung der Koppelstange 76 durch eine geringfügige Modifikation ersetzt werden.

Im übrigen läßt sich die Erfindung auch bei scherenartigen Griffteilen vorteilhaft einsetzen.

## Patentansprüche

1. Handgriff für ein medizinisches Rohrschaftinstrument, mit zumindest einem beweglichen Griffteil (22) und mit einem Kopplungsteil (32), durch das ein Rohrschaft (12) mit dem Handgriff (20) verbunden oder verbindbar ist, wobei das bewegliche Griffteil (22) mit einem in Richtung einer Längsachse (52) des Rohrschafts (12) axial beweglichen Kraftübertragungselement (18) verbunden oder verbindbar ist, wobei eine Bewegung des zumindest einen beweglichen Griffteils (22) in eine axiale Bewegung des Kraftübertragungselements (18) umgesetzt wird, und wobei das Kopplungsteil (32) um eine quer zur Längsachse (52) des Rohrschafts (12) verlaufende Drehachse (50) relativ zu einer Handgriffachse (54) verschwenkbar und arretierbar ist, **dadurch gekennzeichnet, daß** ein als zweiarmiger Hebel ausgebildetes Steuerelement (64) vorgesehen ist, dessen einer Hebelarm mit dem beweglichen Griffteil (22) und dessen anderer Hebelarm mit dem Kraftübertragungselement (18) in Verbindung steht, derart, daß eine Bewegung des beweglichen Griffteils (22) eine Drehung des Steuerelements (64) bewirkt, die vom Steuerelement (64) in eine axiale Bewegung des Kraftübertragungselements (18) umgesetzt wird.

2. Handgriff nach Anspruch 1, **dadurch gekennzeichnet, daß** die Drehachse (50) des Kopplungsteils mit einer Drehachse des Steuerelements (64) zusammenfällt.

3. Handgriff nach Anspruch 1 oder 2, **dadurch gekennzeichnet, daß** das Steuerelement (64) mit dem Kopplungsteil (32) verbunden ist und mit diesem bei einem Verschwenken des Kopplungsteils (32) zum Abwinkeln des Handgriffs (20) von dem Rohrschaft mit verschwenkt wird, wobei zum Verschwenken des Steuerelements (64) die Verbindung zwischen dem beweglichen Griffteil (22) lösbar und nach dem Verschwenken wieder herstellbar ist.

4. Handgriff nach einem der Ansprüche 1 bis 3, **dadurch gekennzeichnet, daß** das zumindest eine bewegliche Griffteil (22) mit einem ersten Hebelarm (74) eines zweiarmigen Hebels (66) verbunden ist, dessen anderer Hebelarm (68) mit dem zweiten Hebelarm des Steuerelements (64) verbunden ist, wobei eine Drehachse des Hebels (66) mit der Drehachse (50) des Kopplungsteils (32) zusammenfällt.

5. Handgriff nach Anspruch 4, **dadurch gekennzeichnet, daß** der zweite Hebelarm (68) des Hebels (66) über einen Zapfen (70) mit dem Steuerelement (64) verbunden ist, wobei in dem Steuerelement (64) mehrere auf einem Teilkreis angeordnete Bohrungen (72) vorhanden sind, mit denen der Zapfen (70) je nach Schwenkstellung des Kopplungsteils (32) in Eingriff bringbar ist.

6. Handgriff nach einem der Ansprüche 1 bis 5, **dadurch gekennzeichnet, daß** das Kopplungsteil (32) mittels eines Rastmechanismus (60) in mehreren Schwenkstellungen in einem Gesamtwinkelbereich von bis zu etwa 240° arretierbar ist.

7. Handgriff nach Anspruch 6, **dadurch gekennzeichnet, daß** der Rastmechanismus (60) eine Rastnase (58) aufweist, die mit Aussparungen (56) des Kopplungsteils (32) in Eingriff bringbar ist, wobei der Rastmechanismus (60) weiterhin einen Druckknopf (62) aufweist, so daß durch Drücken des Druckknopfes (62) die Rastnase (58) mit der jeweiligen Aussparung (56) außer Eingriff bringbar ist.

8. Handgriff nach Anspruch 5 und nach Anspruch 7, **dadurch gekennzeichnet, daß** der Zapfen (70) mit dem Druckknopf (62) verbunden und durch Drücken des Druckknopfes (62) mit der jeweiligen Bohrung (72) außer Eingriff bringbar ist.

9. Handgriff nach einem der Ansprüche 1 bis 8, **dadurch gekennzeichnet, daß** der zweite Hebelarm des Steuerelements (64) eine als Steuerkurve ausgebildete Aussparung (82) aufweist, in der ein mit dem Kraftübertragungselement (18) verbundener bzw. verbindbarer Stift (84) axial geführt ist.

10. Handgriff nach einem der Ansprüche 1 bis 8, **dadurch gekennzeichnet, daß** das Kraftübertragungselement (18) über einen Hebel mit dem Steuerelement (64) verbunden ist.

11. Handgriff nach Anspruch 9 oder 10, **dadurch gekennzeichnet, daß** das Kopplungsteil (32) eine Aufnahme (88) für ein proximales Ende des Kraftübertragungselements (18) aufweist, wobei die Aufnahme (88) axial beweglich ist und mit dem Stift (84) bzw. dem Hebel verbunden ist.

12. Handgriff nach Anspruch 11, **dadurch gekennzeichnet, daß** die Aufnahme (88) einen Verrastmechanismus (90) zum lösbaren Verbinden des. Kraftübertragungselements (18) mit dem Kopplungsteil (32) aufweist.

13. Handgriff nach einem der Ansprüche 1 bis 12, **dadurch gekennzeichnet, daß** das Kopplungsteil (32) eine Aufnahme für den Rohrschaft (12) zum lösbaren Verbinden des Rohrschafts (12) mit dem Kopplungsteil aufweist.

14. Medizinisches Rohrschaftelement, mit einem Rohrschaft (12), mit zumindest einem beweglichen Werkzeug (14, 16) am distalen Ende des Rohrschafts (12), mit einem axial in dem Rohrschaft (12) beweglich angeordneten Kraftübertragungselement (18) zum Betätigen des zumindest einen beweglichen Werkzeugs (14, 16), **gekennzeichnet durch** einen Handgriff (20) nach einem der Ansprüche 1 bis 12.

15. Rohrschaftinstrument nach Anspruch 14, **dadurch gekennzeichnet, daß** der Rohrschaft (12) lösbar mit dem Handgriff (20) verbunden ist.

16. Rohrschaftinstrument nach Anspruch 14 oder 15, **dadurch gekennzeichnet, daß** das Kraftübertragungselement (18) lösbar mit dem Handgriff (20) verbunden ist.

17. Rohrschaftinstrument nach einem der Ansprüche 14 bis 16, **dadurch gekennzeichnet, daß** das Kraftübertragungselement (18) lösbar mit dem Rohrschaft (12) verbunden ist.

## Claims

1. A handle for a medical tubular shaft instrument, comprising at least one moveable grip element (22) and a coupling portion (32) through which the tubular shaft (12) is connected or can be connected to the handle (20), wherein the moveable grip element (22) is connected or can be connected to a force transmission element (18) axially moveable in direction of a longitudinal axis (52) of the tubular shaft (12), wherein motion of the at least one moveable grip element (22) is translated into an axial motion of the force transmission element (18), and wherein the coupling portion (32) is pivotal and lockable relative to a handle axis (54) about a pivot axis (50) running transversely to the longitudinal axis (52) of the tubular shaft (12), **characterized in that** a control element (64) formed as a double-arm lever is provided whose one lever arm is connected to the moveable grip element (22) and whose other lever arm is connected to the force transmission element (18), such that motion of the moveable grip (22) element causes a rotation of the control element (64), which the control element (64) translates into an axial movement of the force transmission element (18).

2. The handle of claim 1, **characterized in that** the pivot axis (50) of the coupling portion coincides with a pivot axis of the control element (64).

3. The handle of claim 1 or 2, **characterized in that** the control element (64) is connected to the coupling portion (32) and rotates with the coupling portion when pivoted to form an angle between the handle (20) and the tubular shaft, wherein the connection between the moveable grip element (22) for rotating the control element (64) is releasable and can be re-established after rotation.

4. The handle of one of claims 1 through 3, **characterized in that** the at least one moveable grip element (22) is connected to a first lever arm (74) of a double-arm lever (66), whose other lever arm (68) is connected to the second lever arm of the control element (64), wherein a pivot axis of the lever (66) coincides with the pivot axis (50) of the coupling portion (32).

5. The handle of claim 4, **characterized in that** the second lever arm (68) of the lever (66) is connected via a pin (70) to the control element (64), wherein several holes (72) are provided in the control element (64) arranged on a partial circular section, with which holes (72) the pin is engageable depending on the pivot position of the coupling portion (32).

6. The handle of one of claims 1 through 5, **characterized in that** the coupling portion (32) is lockable in several pivot positions by means of a locking mechanism (60) in a total angular region of up to about 240°.

7. The handle of claim 6, **characterized in that** the locking mechanism (60) comprises a locking nose (58) engageable with the recesses (56) of the coupling portion (32), wherein the locking mechanism (60) further comprises a button (62), so that by depressing the button (62) the locking nose (58) is disengageable with the respective recess (56).

8. The handle of claim 5 and claim 7, **characterized in that** the pin (70) is connected to the button (62) and is disengageable with the respective hole (72) by depressing the button (62).

9. The handle of one of claims 1 through 8, **characterized in that** the second lever arm of the control element (64) comprises a recess (82) formed as a guide slot in which a pin (84) connected to or connectable to the force transmission element (18) is axially guided.

10. The handle of one of claims 1 through 8, **characterized in that** the force transmission element (18) is connected to the control element (64) through a lever.

11. The handle of claim 9 or 10, **characterized in that** the coupling portion (32) comprises a seat (88) for a proximal end of the force transmission element (18) and wherein the seat (88) is axially moveable and is connected to the pin (84) or the lever.

12. The handle of claim 11, **characterized in that** the seat (88) comprises a locking mechanism (90) for releaseable connection of the force transmission element (18) to the coupling portion (32).

13. The handle of one of claims 1 through 12, **characterized in that** the coupling portion (32) comprises a seat for the tubular shaft (12) for releaseable connection of the tubular shaft (12) to the coupling portion.

14. A medical instrument, comprising a tubular shaft (12), at least one moveable tool (14, 16) at the distal end of the tubular shaft (12) and a force transmission element (18) arranged to be axially moveable in the tubular shaft (12) for actuating the at least one moveable tool (14, 16), **characterized by** a handle (20) according to one of claims 1 through 12.

15. The medical instrument of claim 14, **characterized in that** the tubular shaft (12) is releaseably connected to the handle (20).

16. The medical instrument of claim 14 or 15, **characterized in that** the force transmission element (18) is releaseably connected to the handle (20).

17. The medical instrument of one of claims 14 through 16, **characterized in that** the force transmission element (18) is releaseably connected to the tubular shaft (12).

## Revendications

1. Poignée pour un instrument médical à tige tubulaire, comportant au moins une partie de préhension mobile (22) et une partie d'accouplement (32), par laquelle une tige tubulaire (12) est reliée ou peut être reliée à la poignée (20), la partie de préhension mobile (22) étant reliée ou pouvant être reliée à un élément de transmission des forces (18), déplaçable axialement dans la direction d'un axe longitudinal (52) de la tige tubulaire (12), un mouvement de la ou des parties de préhension mobiles (22) étant converti en un mouvement axial de l'élément de transmission des forces (18), et la partie d'accouplement (32) pouvant pivoter et être bloquée autour d'un axe de rotation (50), s'étendant perpendiculairement à l'axe longitudinal (52) de la tige tubulaire (12), par rapport à un axe de poignée (54), **caractérisée en ce qu'**il est prévu un élément de commande (64) conformé en levier à deux bras, dont un bras de levier est en liaison avec la partie de préhension mobile (22) et dont l'autre bras de levier est en liaison avec l'élément de transmission des forces (18), de manière qu'un mouvement de la partie de préhension mobile (22) provoque une rotation de l'élément de commande (64), qui est convertie par l'élément de commande (64) en un mouvement axial de l'élément de transmission des forces (18).

2. Poignée selon la revendication 1, **caractérisée en ce que** l'axe de rotation (50) de la partie d'accouplement coïncide avec un axe de rotation de l'élément de commande (64).

3. Poignée selon la revendication 1 ou 2, **caractérisée en ce que** l'élément de commande (64) est relié à la partie d'accouplement (32) et est pivoté avec cette dernière, lors d'un pivotement de la partie d'accouplement (32), pour dévier la poignée (20) de la tige tubulaire, la liaison entre la partie de préhension mobile (22) pouvant être supprimée, pour le pivotement de l'élément de commande (64), et rétablie après le pivotement.

4. Poignée selon l'une des revendications 1 à 3, **caractérisée en ce que** la ou les parties de préhension mobiles (22) est ou sont reliées à un premier bras de levier (74) d'un levier (66) à deux bras, dont l'autre bras de levier (68) est relié au deuxième bras de levier de l'élément de commande (64), un axe de rotation du levier (66) coïncidant avec l'axe de rotation (50) de la partie d'accouplement (32).

5. Poignée selon la revendication 4, **caractérisée en ce que** le deuxième bras de levier (68) du levier (66) est relié à l'élément de commande (64), par un axe (70), dans l'élément de commande (64) étant prévus plusieurs trous (72) disposés sur un cercle partiel, avec lesquels l'axe (70) peut être amené en prise, suivant la position de pivotement de la partie d'accouplement (32).

6. Poignée selon l'une des revendications 1 à 5, **caractérisée en ce que** la partie d'accouplement (32) peut être bloquée, au moyen d'un mécanisme à cran d'arrêt (60), dans plusieurs positions de pivotement, sur une plage angulaire totale pouvant aller jusqu'à environ 240°.

7. Poignée selon la revendication 6, **caractérisée en ce que** le mécanisme à cran d'arrêt (60) comporte un ergot d'arrêt (58) qui peut être amené en prise avec des découpes (56) de la partie d'accouplement (32), le mécanisme à cran d'arrêt (60) comportant en outre un bouton-poussoir (62), ce qui fait que lorsque l'on enfonce le bouton-poussoir (62), l'ergot d'arrêt (58) peut être désengagé de la découpe (56) correspondante.

8. Poignée selon la revendication 5 et la revendication 7, **caractérisée en ce que** l'axe (70) est relié au bouton-poussoir (62) et peut être désengagé du trou (72) correspondant, par enfoncement du bouton-poussoir (62).

9. Poignée selon l'une des revendications 1 à 8, **caractérisée en ce que** le deuxième bras de levier de l'élément de commande (64) comporte une découpe (82), formée en tant que courbe de commande, dans laquelle est guidée axialement une cheville (84) qui est reliée ou peut être reliée à l'élément de transmission des forces (18).

10. Poignée selon l'une des revendications 1 à 8, **caractérisée en ce que** l'élément de transmission des forces (18) est relié à l'élément de commande (64), par un levier.

11. Poignée selon la revendication 9 ou 10, **caractérisée en ce que** la partie d'accouplement (32) comporte un logement (88) pour une extrémité proximale de l'élément de transmission des forces (18), le logement (88) étant déplaçable axialement et relié à la cheville (84), respectivement au levier.

12. Poignée selon la revendication 11, **caractérisée en ce que** le logement (88) comporte un mécanisme d'encliquetage (90) pour la liaison amovible de l'élément de transmission des forces (18) avec la partie d'accouplement (32).

13. Poignée selon l'une des revendications 1 à 12, **caractérisée en ce que** la partie d'accouplement (32) comporte un logement pour la tige tubulaire (12), en vue de la liaison amovible de la tige tubulaire (12) avec la partie d'accouplement.

14. Elément médical à tige tubulaire, comportant une tige tubulaire (12), avec au moins un outil déplaçable (14, 16) à l'extrémité distale de la tige tubulaire (12), comportant un élément de transmission des forces (18), disposé déplaçable axialement dans la tige tubulaire (12), pour l'actionnement du ou des outils déplaçables (14, 16), **caractérisé par** une poignée (20) selon l'une des revendications 1 à 12.

15. Instrument à tige tubulaire selon la revendication 14, **caractérisé en ce que** la tige tubulaire (12) est reliée de manière amovible à la poignée (20).

16. Instrument à tige tubulaire selon la revendication 14 ou 15, **caractérisé en ce que** l'élément de transmission des forces (18) est relié de manière amovible à la poignée (20).

17. Instrument à tige tubulaire selon l'une des revendications 14 à 16, **caractérisé en ce que** l'élément de transmission des forces (18) est relié de manière amovible à la tige tubulaire (12).
